# EUROPEAN PATENT APPLICATION

(11) **EP 1 357 192 A1**
(43) Date of publication of application: **29.10.2003**
(21) Application number: 02076574.9
(22) Date of filing: 22.04.2002
(51) Int. Cl.: C12P 7/26, A23L 1/23

(54) **Biosynthesis of hydroxyketones**

(71) Applicant: SOCIETE DES PRODUITS NESTLE S.A., 1800 Vevey (CH)
(72) Inventor: Kurniadi, Toshinari, 1510 Moudon (CH); Belrhlid, Rachid, 1066 Epalinges (CH); Berger, Ralf Günter, 30455 Hannover (DE); Juillerat, Marcel Alexandre, 1000 Lausanne 26 (CH); Fay, Laurent Bernard, 74500 Evian (FR)
(74) Representative: Wavre, Claude-Alain

(57) **Abstract**

The present invention relates to a process for the generation of hydroxyketones consisting in subjecting an aldehyde, preferable acrolein, to bioconversion with a micro-organism providing pyruvate decarboxylase activity.

## Description

The present invention relates to the field of bio-synthesis of aroma-active hydroxyketones and derivatives.

Hydroxyketones are very useful in both food and pharmaceutical industries. Hydroxyketones may serve as precursors for the preparation of bio-active compounds. In the food area, hydroxyketones may be used as building blocks for other flavour active compounds, that is to say pyrazines and derivatives thereof but may also be used as flavourants per se.
JP 5316959 discloses the use of hydroxyketones to improve the aroma, to give rich taste and to modify the acidic and the salty taste of foodstuffs by adding a specific alpha-hydroxyketone derivative in a specified ratio. In such case the compound is obtained by thermally decomposing a saccharide and subsequently isolating and purifying the thermally decomposed material.
DE 2217483 discloses a process for the manufacture of hydroxyketones by treatment of an alcohol by mercury and strong acid.
US 5831097 also discloses a chemical synthesis process for manufacturing hydroxyketones based on the reaction of two aldehydes in the presence of a catalyst.
Thus, most of the manufacturing processes of hydroxyketones are chemical ones and are thus cumbersome and expensive.
A particular hydroxyketone, L-phenylacetylcarbinol (L-PAC) may be produced by decarboxylation of pyruvate by the enzyme pyruvate decarboxylase in the presence of benzaldehyde (Oliver & al, 1999, Adv. Microb. Phys. 41, 1-45). L-PAC is used as a substrate for manufature of ephedrine derivatives which are two important pharmaceutical alkaloids.
Hydroxyketones are flavourant molecules occuring in foodstuffs and serve as aroma precursors as well. Different hydroxyketones have been described as aroma active and identified in flower, cheese and wine, for example (Watanabe & al, 1988, Proceedings of the 10^{th} international congress of essentials oils, fragrances and flavours, 425-437. Moio & al, 1993, Ital. J. Food Sci., 5, 215-225. Brock & al, 1984, Am. J. Enol. Vitic., 35, 151-155).
Neuser & al (J. Agric. Food Chem., 48, 6191-6195) highlighted the occurrence and generation of several odorous hydroxyketones in sherry and soy sauce. It was also reported that the wild type Zygosaccharomyces bisporus CBS 702 produces hydroxyketones from amino acid precursors after transamination to the corresponding 2-oxo acids. The key enzyme for decarboxylation and C-C bond formation, pyruvate decarboxylase (PDC) was examined for its specificity features. It was disclosed that starting with a 2-oxo-acid and an aldehyde, a side activity of pyruvate decarboxylase resulted in the formation of alpha-hydroxy ketones in an acyloin-type condensation reaction (Neuser & al, 2000, Z.Naturforsch., 55c, 560-568).

The use of purified enzymes requiring cofactors is sometimes cumbersome and expensive as well. Moreover, the use of pure enzyme involves a further purification step in order to recover the reaction products. Finally, the use of starting materials like oxo-acids and aldehydes are alo complicating factors in order to produce aroma-active hydroxyketones.

Due to the high interest and high application potencial in the food area for hydroxyketones compounds, there remains a need for a simple, quick, natural, cheap and food grade method for preparing hydroxyketones.

Accordingly, the present invention provides a process for the biogeneration of aroma-active hydroxyketones of the formula :

CH₃-CO-HCOH-R and CH3-HCOH-CO-R

characterized by the fact that at least one compound A selected from the group comprising :

R-CHO

is subjected to bioconversion in a medium in the presence of a micro-organism providing a pyruvate decarboxylase activity and in which radical R is selected from the group comprising H, alkyl or alkenyl group from C1 to C8 linear or ramified.
In the present description the term "aroma-active" means that the hydroxyketones obtained according to the present process may be considered as flavouring compounds in the sense that they are have a pleasant aroma profile per se, but that they can serve as aroma precursors for generating other kind of aromatic compound, such as pyrazines for exemple.
The micro-organism used for the bioconversion may be resting cells, disrupted or intact ones. The micro-organism used for the bioconversion may be in the form of an extract, a powder or a cream solution. Preferably, the micro-organism is a fresh solution of grown micro-organism, or of up 4 to 8 days, kept in the refrigerator.
In one preferred embodiment, the micro-organism is selected from the group comprising bacteria, yeasts, moulds and fungus. The preferred bacteria are selected from the group comprising Zymomonas, Lactobacillus and Bacillus. The preferred moulds and fungus are selected from the group comprising Neurosporae and Aspergillae. The preferred yeasts are selected from the group comprising Saccharomyces, Debaromyces, Candida, Pichia, Schizosaccharomyces and Zygosaccharomyces. More preferable, the yeast is baker's yeast, Saccharomyces cerevisiae.
R is preferably an ethylen group and compound A is therefore acrolein that will lead upon bioconversion with pyruvate in the presence of baker's yeast cells to 3-hydroxy-4-pentene-2-one. The bioconverion may also lead to the isomer 2-hydroxy-4-penten-3-one, depending upon reactants relative aboundance. This bioconversion is achieved according to the following route : 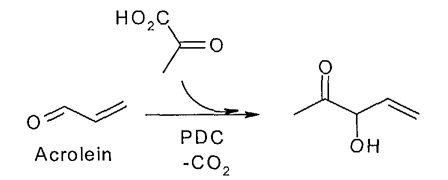 The activity of the pyruvate decarboxylase involves the decarboxylation of the pyruvate which is a metabolite of the micro-organism. The pyruvate is present in the medium because of the metabolim of the micro-organism ; however, in order to facilitate and enhance the bioconversion yield is may be preferable to add pyruvate in the bioconversion medium. Moreover, during the bioconversion of acrolein it has been noticed that no production of phenyl ethanol occurred. This means that acrolein is competitive to phenyl pyruvate (an other metabolite of the micro-organism) and that the bioconversion is achieved thanks to phenyl pyruvate decarboxylase activity as well.
The concentrations of the starting products in the medium, e.g. compound A and pyruvate, may be in the range of 0.5 mM to 500 mM for A and in the range of from 0.5 to 1000 mM for pyruvate. As said before, the pyruvate in the medium may either come from the metabolism of the micro-organism or be added in the medium or both.

Regarding the respective quantities of the starting products A and pyruvate, they can be such that the molar ratio A:pyruvate may vary from 1:2000 to 500:1. Optimal ration A.pyruvate will depend on the relative aboundance of the desired isomer.
In the case of the use of acrolein as compound A, the more pyruvate is be present in the medium, compared to acrolein, the more 3-hydroxy-4-pentene-2-one is produced and alternatively the less pyruvate is present in the medium compared to acrolein, the more 2-hydroxy-4-pentene-3-one is produced. One has to keep in mind that 3-hydroxy-4-pentene-2-one is however preferentially formed.
The compound A may be added in the medium completely, i.e. in one step, in the medium or may be advantageously added in the bioconversion medium by mean of fed-batch mode. Such progressive addition of compound A may allow to reduce the inhibitory effect of such compound on the formation of hydroxyketones.
Generally the micro-organism solution is used as from 1 to 30% w/v of micro-organism cells in the reaction medium.

The bioconversion may be carried out under aerobic or anaerobic conditions during from 15 minutes to 48 hours, preferably from 1 to 5 hours and at a pH of from 4 to 8, preferably from 5 to 7, more preferably about 6.
The cells of the micro-organism may be immobilized on a solid support such as bead or membrane by appropriate means such as encapsulation for example.
The temperature of the bioconversion may range from 4 to 45°C, preferably from 20 to 35°C.

The raction medium may be water or buffer such as citrate phosphate or Tris buffers of concentration between 50 to 200 mM, for example. The reaction medium may also comprise ethanol or methanol in a concentration of up to 5% w/v which may serve as increasing the bioconversion yield.

Some co-factors of the decarboxylation like Thiamine pyro phosphate (TPP) and Mg⁺⁺ are generally present in the cells of the micro-organism and therefore in the bioconversion medium. Such compounds may also be added in the medium in order, to enhance the yield or accelerate the bioconversion rate. Mg⁺⁺ may be added in the medium as MgCl₂ or MgSO₄ in order to obtain a concentration of free Mg⁺⁺ in the range of from 0.1 mM to 20 mM. TPP may be added in them medium in the range of from 0.1 mM. to 10 mM.

A further aspect of the present invention concerns a process for the manufacture of flavouring composition consisting in recovering flavour compounds from the bioconversion medium containing aroma-active hydroxyketones.
The bioconversion medium obtained by the process according to the present invention using acrolein as compound A contains hydroxyketones, however it also contains other kind of flavour compounds selected from the group comprising : hydroxyketones, gamma-penta-lactone, acetoine and decalactone.

The bioconversion medium may be submitted to a extraction step in order to recover the flavour compounds contained. Thus, these flavours compounds may be recovered by means of two-phase liquid extraction using organic solvant. Accordingly, an organic solvant may be mixed with the medium either after or during the bioconversion allowing the migration of the formed flavour compounds from the medium to the solvant. The suitable organic solvants for performing the extraction may be hexane, pentane, dichloromethane or diethyl ether for example, used alone or in combination.
Alternatively, the recovering of the flavour compounds may be achieved in situ by means of adsorption on a solid phase contacted with the bioconversion medium. Such recovering by adsorption on a solid phase may be performed by contacting beads or membranes with the bioconversion medium. These beads or membranes may be of a material that exhibits good adsorption affinity for the flavour compounds of the medium. Such kind of material may be selected from the group comprising cyclodextrine or amberlite for example.

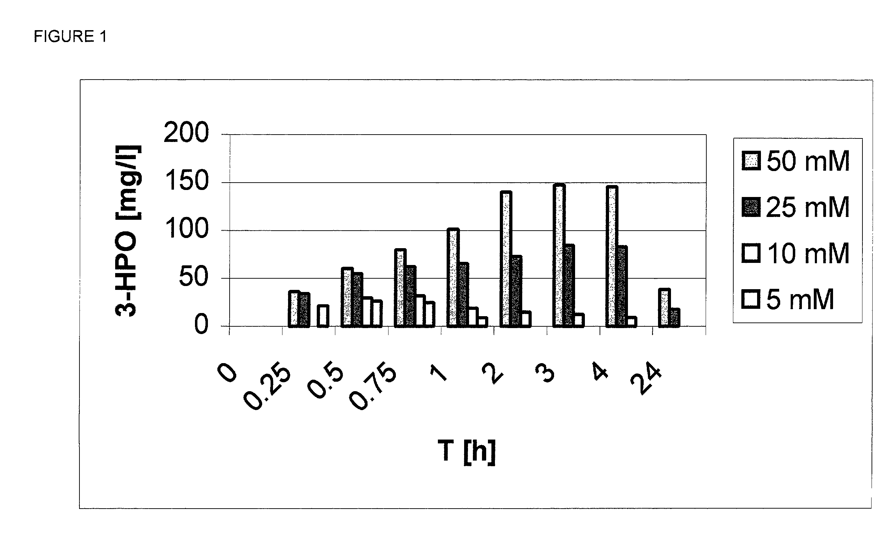

Figure 1 shows the production of 3-hydroxy-4-pentene-2-one (3HPO) in mg/l obtained after different incubation times T (in hours) with different acrolein concentration 5,10, 25 and 50 mM.

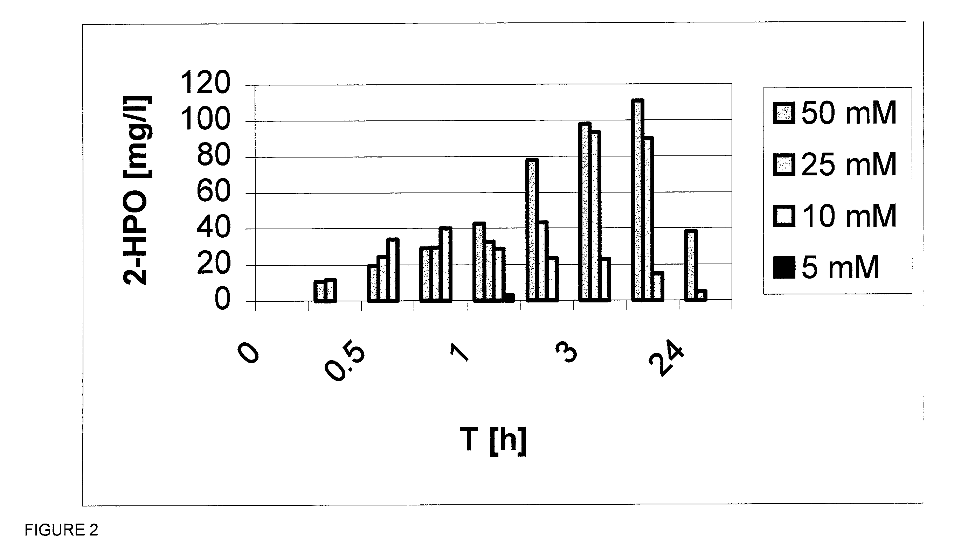

Figure 2 shows the production of 2-hydroxy-4-pentene-3-one (2HPO) in mg/l obtained after different incubation times T (in hours) with different acrolein concentration 5,10, 25 and 50 mM.

The processes according to the present invention and the differents embodiments are hereby described by way of examples.

### MATERIAL AND METHODS

Dried baker's yeast for biotransformation studies was purchased by Hefe Schweiz. All chemicals and PDC were from Sigma Aldrich Chemical Co.

### BIOCONVERSION

Bioconversion studies were carried out with commercially-available dried baker's yeast in shaking flasks equipped with an electrode and a magnetic stirrer (500 rpm). Where necessary, the flask was adjusted to a certain temperature using an oil bath and the pH was automatically maintained using a Metrohm pH-stat device (Impulsomat 614). After the equilibrum of the latter physical parameters was reached, all precursors were separately added. A kinetic study was performed by collecting aliquots, centrifugating them and further analytical treatment of the supernatant.

### GC AND GC-MS ANALYSES

GC-analyses were performed on a Carbo Erba 8000 series GC equipped with an automatic cold on-column injector, a flame ionization detector (FID) and a DB-Wax capillary column (30m x 0.25mm, film thickness 0.25 mm). The carrier gas was helium (80kPa), make up-gas for the FID was nitrogen (40kPa). The injected volume was between 0.5 and 2.5 µl. The oven temperature profile was at follows: initial temperature: 30°C, program rate I: 40°C/min - final temperature I: 60 °C, 1 min at 60 °C, program rate II: 4°C/min, final temperature II: 200 °C, 20 min at 200 °C.

GC-MS analyses were performed on a Finnigan MAT-8430 mass spectrometer combined with an HP 5890 gas chromatograph using the same conditions as described above. The MS-EI spectra were generated at 70 eV and MS-CI at 150 eV with ammonia as reagent gas.

### QUANTITATION OF HYDROXYKETONES

The aqueous phases were extracted twice with one volume of distilled diethyl ether. Before extraction acetol as internal standard was added. We assumed that the FID response factor of 3-hydroxy-4-pentene-2-one was 4 times greater than that of acetol.

### RESULTS

### PRODUCTION OF 3-HYDROXY-PENT-4-ENE-2-ONE BY PDC

With PDC from *Saccharomyces cerevisiae*, and pyruvate and acrolein both at initial concentrations of 118 mM, we observed after 48 hours the production of 3-hydroxy-pent-4-ene-2-one near the detection limit which was proved by GC-MS (Table 1).

**Table 1.**

| MS data of 3-hydroxy-4-pentene-2-one. | | |
|---|---|---|
| No. | Acyloin | MS, m/e (%) |
| 16 | 3-Hydroxy-4-pentene-2-one | 100(93), 98(28), 85(13), 71(72), 57(94), 43(100) |

### OPTIMIZATION OF PDC REACTION

The optimization of a bioconversion process is dependent on an array of parameters, including the concentration of substrates used, the physical conditions for production and the medium composition, all of which when correctly combined create conditions conducive to higher production yields. Studies into the optimization of production of 3-hydroxy-4-pentene-2-one are described below.

### Kinetics of enzymatic reaction and effect of substrate concentration

It was clear to us that we had to modify the reaction parameters such that the enzymatic hydroxyketone production should be in favour versus chemical acrolein dimerization. In order to simultaneously determine the optimal incubation time, we investigated the time course of the enzymatic reaction for acrolein concentrations ranging from 0.5 mM to 5 mM during 24 hours, while in all essays the pyruvate concentration was kept constant at 118 mM.
In fact, the less acrolein was used, the less acrolein dimer was formed. Furthermore, after two hours, we observed a relatively effective formation of 3-hydroxy-4-pentene-2-one, the optimal conversion rate accounting to approximately 20 % at initial acrolein concentrations of 0.5 mM.
Formation of 3-hydroxy-4-pentene-2-one was always observed during the first two to three hours. After that, no further product was formed, the amounts even slightly decreasing due to possible slow degradation of the product. Conclusively, these results indicate that successive substrate feeding with low acrolein concentrations around 0.5 mM should be investigated.

### Physicochemical conditions and their effect on production of 3-hydroxy-4-pentene-2-one

### Effect of pH on bioconversion

To determine the effect of pH on the efficacy of the bioconversion reaction, a pH range of 3 to 8 was employed to perform further experiments. We observed best conversion rates for 3-hydroxy-4-pentene-2-one at a pH of 6.0, good results were obtained in the range of from 5 to 7 and the extreme values were 4 and 8.

### Effect of temperature on bioconversion

To determine the effect of temperature, bioconversion was performed at 4°, 23° C and 37°. We observed that after two hours, at 4°C, the conversion yield was tremendously decreased in comparison to that at 23 °C leading to about 0.02 mM of 3-hydroxy-4-pentene-2-one. At 37 °C the conversion rate was slightly increased after 2 hours compared with that at 23 °C which accounts to 117 % of that at 23°C.

### Effect of buffer, ion strength and enzyme quantity on production of 3-hydroxy-4-pentene-2-one

No improvement of hydroxyketone conversion rates was observed when modifying citrate buffer concentrations between 50 mM, 100 mM and 200 mM. Using 100 mM phosphate buffer instead of 100 mM citrate buffer even reduced the conversion rates by a factor of two. However, increasing the PDC quantity by a factor of two at acrolein concentrations of 4.8 mM, led to approximately 1.5 fold enhancement of conversion rates.

### PRODUCTION OF 3-HYDROXY-4-PENTENE-2-ONE, 2-HYDROXY-4-PENTENE-3-ONE AND 4-PENTENE-2,3-DIONE BY S. CEREVISISAE

In order to produce greater amounts of 3-hydroxy-4-pentene-2-one, we went on performing bioconversion experiments with whole cells of baker's yeast, instead of using PDC. When using acrolein and pyruvate at 19 mM concentrations and an initial glucose concentration of 4 g/l, in a 100 mM phosphate buffer containing 25 mM MgSO₄, we identified 3-hydroxy-4-pentene-2-one by GC-MS. Interestingly, we further observed the formation of 2-hydroxy-4-pentene-3-one, the corresponding hydroxyketone isomer, as well as 4-pentene-2,3-dione, the corresponding desaturated diketone (Table 2).

**Table 2.**

| MS data of 2-hydroxy-4-pentene-3-one and 4-pentene-2,3-dione. | | |
|---|---|---|
| No. | Product | MS, m/e (%) |
| 17 | 2-Hydroxy-4-pentene-3-one | 100(10), 98(7), 85(2), 71(2), 56(75), 55(72), 45(81), 44(100) |
| 18 | 4-Pentene-2,3-dione | 98(73), 70(2), 55(100), 43(94) |

## Claims

1. Process for the biogeneration of aroma-active hydroxyketones of the formula :
CH₃-CO-HCOH-R and CH3-HCOH-CO-R
**characterized by** the fact that at least one compound A selected from the group comprising :
R-CHO
is subjected to bioconversion in a medium in the presence of a micro-organism providing a pyruvate decarboxylase activity and in which radical R is selected from the group comprising H, alkyl or alkenyl group from C1 to C8 linear or ramified.

2. Process according to claim 1 **characterized in that** the micro-organism provides a phenyl pyruvate decarboxylase activity.

3. Process according to claim 1 **characterized in that** pyruvate and/or phenylpyruvate are added in the medium.

4. Process according to claim 1 **characterized in that** co-factors are added in the medium.

5. Process according to claim 4 **characterized in that** the co-factors are selected from the group comprising thiamine-pyro-phosphate and magnesium.

6. Process according to claim 1 **characterized in that** the micro-organism is selected from the group comprising bacteria, yeast, moulds and fungus.

7. Process according to claim 6, **characterized in that** the micro-organism is a yeast selected from the group comprising yeast of the genus Saccharomyces, Debaromyces, Candida, Pichia, Schizosaccharomyces, Zygosaccharomyces.

8. Process according to claim 7, **characterized in that** the micro-organism is baker's yeast, Saccharomyces cerevisiae.

9. Process according to claim 1, **characterized in that** the micro-organism is immobilized.

10. Process according to claim 1, **characterized in that** the compound A is acrolein :
CH2=CH-CHO

11. Process for the manufacture of flavouring composition consisting in recovering flavour compounds from the bioconversion medium according to claim 1.

12. Process according to claim 11, **characterized in that** the flavour compounds are selected from the group comprising : hydroxyketones, gamme-penta-lactone, acetoine, decalactone.

13. Process according to claim 11, **characterized in that** the flavour compounds are recovered by mean of organic solvant extraction.

14. Process according to claim 13, **characterized by** the fact that the solvant is mixed with the medium during the bioconverion allowing the migration of formed flavour compounds from the medium to the solvant.

15. Process according to claim 11, **characterized in that** the recovering of flavour compounds is achieved by mean of adsorption on a solid phase contacted with the bioconversion medium.
